# EUROPEAN PATENT APPLICATION

(11) **EP 4 649 881 A1**
(43) Date of publication of application: **19.11.2025**
(21) Application number: 25176979.0
(22) Date of filing: 16.05.2025
(51) Int. Cl.: A61B 1/00, A61B 1/05, A61B 1/267

(54) **ADJUSTABLE VIDEOLARYNGOSCOPE**

(30) Priority: 16.05.2024 DE 102024001595
(71) Applicant: Universitätsklinikum Hamburg-Eppendorf (UKE), 20246 Hamburg (DE)
(72) Inventor: PETZOLDT, Martin, 20257 Hamburg (DE); WISCHNEWSKI, Dennis, 22527 Hamburg (DE)
(74) Representative: Eisenführ Speiser

(57) **Abstract**

The invention relates to a laryngoscope for assisting a user in endotracheal intubation of a patient by laryngoscopy. The laryngoscope comprises a) a handle (10) for the user to hold the laryngoscope, b) a blade (20) adapted to extend from the handle into a pharyngeal space of the patient when the laryngoscope is held by the user, and c) a camera (32) for imaging at least a portion of the pharyngeal space from a position on the blade when the blade extends into the pharyngeal space. The laryngoscope further comprises mechanics operable by the user while holding the laryngoscope, and the blade comprises an adjustable blade tip (21), wherein the blade tip (21) is adjustable by the user via the mechanics. A laryngoscope of this kind allows for safer endotracheal intubations.

## Description

### FIELD OF THE INVENTION

The invention relates to a laryngoscope for assisting a user in endotracheal intubation of a patient by laryngoscopy.

### BACKGROUND OF THE INVENTION

Endotracheal intubation, also referred to as tracheal intubation or just intubation, is considered to be a difficult procedure, wherein mistakes in carrying out an intubation can cause serious harm to a patient. A main challenge in an intubation is to obtain a good view on the glottis of the patient by laryngoscopy. Since the view on the glottis is usually impaired by the epiglottis particularly in anaesthetised patients, a laryngoscope is often used to apply a pressure on the hyoepiglottic ligament in the vallecular and thereby cause the epiglottis to move upward. Nevertheless, the view on the glottis obtained in this way often still does not suffice to carry out a safe intubation. With prior art laryngoscopes, endotracheal intubation is still one of the leading causes of anaesthesia-associated complications.

### SUMMARY OF THE INVENTION

It is an object of the present invention to allow for safer endotracheal intubations.

The invention relates to a laryngoscope for assisting a user in endotracheal intubation of a patient by laryngoscopy, the laryngoscope comprising a handle for the user to hold the laryngoscope, a blade adapted to extend from the handle into a pharyngeal space of the patient when the laryngoscope is held by the user, and a camera for imaging at least a portion of the pharyngeal space from a position on the blade when the blade extends into the pharyngeal space. The laryngoscope further comprises mechanics operable by the user while holding the laryngoscope, and the blade comprises an adjustable blade tip, wherein the blade tip is adjustable by the user via the mechanics.

While the blade of the laryngoscope can be used to obtain a direct view into the pharyngeal space, the camera allows for an additional, indirect view into the same. This can provide the user with additional visual information and thereby assist him or her in carrying out the intubation. In particular, by operation of the mechanics, the adjustable blade tip can be adjusted by the user to apply pressure to the hyoepiglottic ligament for lifting the epiglottis and thereby obtaining a better view on the glottis. The direct view on the glottis obtainable in this way and the indirect view corresponding to an image obtainable by the camera, both of which may not be sufficient on their own, can complement each other to allow the user to carry out an intubation safely.

The camera may comprise a lens arranged on the blade for imaging the portion of the pharyngeal space to be imaged, and illumination means for illuminating the portion of the pharyngeal space to be imaged. The illumination means may be arranged on the blade as well. Furthermore, the camera may comprise a digital image sensor arranged below the lens, wherein the camera may be configured to transmit a video of the pharyngeal space to a monitor. The camera may be an endoscope camera, for instance.

Preferentially, the laryngoscope comprises an actuating element on the handle or on a transition between the handle and the blade, by which the user can control the mechanics and thus adjust the blade tip via one or more adjustment joints. The one or more adjustment joints may correspond to one or more hinges, for instance, i.e., the one or more adjustment joints may comprise one or more hinge joints. The actuating element may also be referred to as an actuation element or as a control element. The one or more adjustment joints may be regarded as one or more joints for adjusting the blade tip. An adjustment of the blade tip may refer to an angulation of the blade tip. With only a single adjustment joint, the blade tip may be the only adjustable part of the blade, whereas with more than one adjustment joint also one or more proximal parts of the blade, i.e. blade parts proximal from the blade tip, may be adjusted, or angulated. Terms like "proximal(ly)" and "distal(ly)" are used herein to indicate relative positions with respect to a body of the laryngoscope and preferentially not a patient's anatomy, wherein a proximal end of the body of the laryngoscope may be formed by the handle, such as by a bottom of the handle, and a distal end of the body of the laryngoscope may be formed by the blade tip.

The actuating element may preferentially be a mechanical actuating element. For instance, the actuating element may be a lever, in which case a position of the lever may indicate a degree to which the blade tip is adjusted. To give a further example, the actuating element may be an adjustment wheel, in which case a position of the wheel may indicate a degree to which the blade tip is adjusted. The position of the wheel may particularly refer to an angular position of the wheel. An operation of the lever or the adjustment wheel, i.e. a change in the position of the lever or the adjustment wheel, may translate into a mechanical force causing the blade tip to be adjusted, such as by causing the one or more adjustment joints to change the angular position.

It may be preferred that the mechanics connect the actuating element to the blade tip via mechanical connection elements. For instance, the mechanics may connect the actuating element to the blade tip via mechanical elements arranged on an exterior of the laryngoscope, i.e., external with respect to other parts of the laryngoscope. This can allow for a simpler construction of the laryngoscope and thus decrease production costs. On the other hand, it is also possible that the mechanics connect the actuating element to the blade tip via mechanical elements arranged internally in the laryngoscope, i.e., internal with respect to other parts of the laryngoscope. This can decrease maintenance needs, since contact between the mechanism and the patient, i.e., tissue and saliva, can be avoided. Internally arranged connection elements can hence also increase hygiene and hence allow for safer intubations in general. The mechanical elements, and particularly the internal mechanical elements, may comprise one or more Bowden cables, for instance.

It may also be preferred that the actuating element is an electronic actuating element. For instance, it may be preferred that the mechanics are electromechanics comprising at least one electric motor electronically connected to the actuating element. The at least one electric motor may be controlled electronically via the electronic actuating element, and may drive an angulation of a respective actuating joint. The at least one electric motor may receive a signal from the actuating element via internal cables, the internal cables being arranged internally in the laryngoscope, i.e., internal with respect to other parts of the laryngoscope. The electronic actuating element may comprise one or more buttons and/or a touchscreen, for instance. Also an adjustment wheel as indicated above could be used as an electronic actuating element, such as if an angular position of the wheel triggers a corresponding electronic signal.

When the mechanics are not operated, the blade may comprise a curvature extending from a proximal blade end, at which the blade meets the handle and which could also be referred to as blade root, to the blade tip. In other words, the blade may comprise a first shape and a second shape, wherein operation of the mechanism may cause the blade to transition from the first shape to the second shape. The blade may already be curved in the first shape from the proximal blade end to the blade tip and optionally including the blade tip. The second shape may differ from the first shape in that the blade tip is angulated to endow the blade with an additional distal curvature. A blade having a curvature independently of an operation of the mechanics by the user can improve a view into the pharyngeal space even without adjusted blade tip and thereby allow the user to initiate an angulation of the blade tip at an optimal position. For instance, the curvature of the blade without adjusted blade tip, i.e., the first shape, may follow a natural anatomic path from a mouth opening to the pharynx. The curvature of the blade without adjusted blade tip may correspond to that of a Macintosh laryngoscope blade, for instance.

The laryngoscope may comprise a base joint at the proximal blade end, via which the blade can be pivoted between a storage position and a usage position, wherein the blade forms a more obtuse angle to the handle in the usage position than in the storage position. In particular, the angle in the usage position may be a right angle and the blade may rest against the handle in the storage position. The blade resting against the handle may also be referred to as the blade abutting the handle. A blade which is pivotal between a storage position and a usage position can allow for safe laryngoscopies without making the laryngoscope impractical for transportation. It is also possible that the blade forms a fixed angle with the handle at the proximal blade end. In other words, the storage position and the usage position may alternatively refer to a same fixed position of the handle. This can allow for an easier construction and less maintenance of the laryngoscope.

It is also an option that the blade is dismountable from the handle for storing the laryngoscope. Thus, for instance, the laryngoscope may be stored and transported in a dismounted state with the handle separated from the blade. For using the laryngoscope, the blade may then be connected to the handle. A plurality of blades with different sizes and shapes may be provided, of which each can be connected to the handle. A user may choose from the different blades depending on a use case, i.e., for instance, depending on an anatomy, particularly a size, of the mouth and the pharyngeal region of the patient.

The blade may be curved in the direction of the handle such that the blade tip forms an angle between 0° and 20° to a plane perpendicular to a longitudinal axis of the handle. The angle may be measured in a usage position of the blade, i.e., in a state of the laryngoscope in which it would be used by the user to carry out the laryngoscopy. Thus, in particular, if the laryngoscope comprises a pivotal blade which can be pivoted between a storage position and a usage position, the angle is not measured in the storage position. An angle between 0° and 20° formed by the blade tip with respect to the plane perpendicular to the longitudinal axis of the handle has been found to allow for a good view into the pharyngeal region even before adjusting the blade tip, and thus for a safe operation of the mechanism of the laryngoscope.

The blade tip is preferentially adjustable in the direction of the handle by an angle between 0° and 70° relative to a proximal part of the blade which is closest to the blade tip. In particular, the blade tip may be adjustable by one of a plurality of user-determinable angles. The proximal part of the blade with respect to which the angle is measured may refer to a most distal part of the blade not belonging to the blade tip, i.e., for instance, a most distal part of the blade which is not adjustable by the angle being measured. Allowing the blade tip to be angulated by an angle between 0° and 70° relative to a proximal part of the blade has been found to allow for a well-balanced pressure to be applied on the hyoepiglottic ligament to obtain a good view on the glottis. Thus, good conditions for a safe intubation can be achieved in this way.

Furthermore, the blade tip preferentially forms a distal end portion of the blade, and the blade preferentially comprises an adjustment joint which is arranged proximally at the distal end portion and whose position is determined through the mechanics operable by the user. The adjustment joint may be actuated by mechanical elements such as Bowden cables or by an electric motor as indicated above, for instance. The adjustment joint may be regarded as a mechanical connection element between the blade tip and a more proximal part of the blade. In principle, a relative angulation of different blade parts, such as the angulation of the blade tip with respect to the more proximal part of the blade, may also be realised differently than with a respective adjustment joint. For instance, the blade may be flexible in a region where it is to be angulated. However, by realising the angulation through an adjustment joint, the blade can be fabricated from robust materials throughout, which can also be easier to disinfect. For instance, the blade and optionally also the handle may be made from a metal or alloy like steel.

Moreover, the blade preferentially comprises at least one further adjustment joint, which divides a region of the blade located proximally of the blade tip into two or more sections adjacent to the respective further adjustment joint, wherein the mechanics operable by the user are configured to further adjust a shape of the blade via a position of the at least one further adjustment joint. For instance, one or more Bowden cables or electric motors may actuate also the one or more further adjustment joints. The at least one further adjustment joint is preferentially configured to tilt the respective two adjacent sections of the blade relative to each other by an angle between 0° and 70°. Thus, the at least one further adjustment joint may have a same range of motion as the adjustment joint effecting the adjustment of the blade tip. It has been found that the one or more further adjustment joints allow for an optimised view into the pharyngeal space as well as an optimised application of pressure on the hyoepiglottic ligament to obtain a yet clearer view of the glottis, thereby further decreasing a risk of harming the patient during intubation.

Thus, the blade preferentially comprises at least two adjustment joints, wherein the two adjustment joints may be hinges. The blade may therefore be movable like a finger and could hence also be referred to as a robot finger. It is further preferred that operation of the mechanics causes the two or more adjustment joints to be jointly adjusted so that the blade tip and one or more sections of the blade proximal to the blade tip are simultaneously angled towards the handle by a respective adjustment angle. This has been found to ease a use of the laryngoscope for the user. In particular, while controlling each adjustment joint separately could allow for a yet more fine-grained adjustment of views into the pharyngeal space and application of pressure on the hyoepiglottic ligament, a joint adjustment of the adjustment joints can be tuned to obtain similarly good views and allow for a similar pressure application while making the laryngoscopy easier for the user. A joint actuation of the two or more adjustment joints may be realized, for instance, by spanning one or more Bowden cables over the two or more adjustment joints or by simultaneously sending control signals to electric motors provided to drive rotations of the angulation joints.

The at least one further adjustment joint may be arranged in a middle to proximal region of the blade. In other words, one adjustment joint may be arranged at a proximal end of a distal blade section corresponding to the blade tip, whereas the at least one further adjustment joint may be arranged more proximally, in the middle or in a yet more proximal region of the blade. However, it is also possible that several adjustment joints are arranged distally from a middle region of the blade. This may lead to a configuration which may be viewed as having a prolonged blade tip with internal mobility, or angulation capability.

The camera may be arranged on a portion of the blade which does not belong to the blade tip. However, it may be preferred that the camera is arranged on the blade tip or, insofar as the laryngoscope comprises at least one further adjustment joint as indicated above, that the camera is arranged at least distally from the at least one further adjustment joint on the blade, so that an orientation of the camera can be changed together with an adjustment of the blade by operation of the mechanics. The camera may be arranged in a distal region, in particular in a distal third, of the blade.

Thus, in particular, the blade may comprise two adjustment joints, wherein the camera is arranged on an intermediate blade portion between the two adjustment joints. An operation of the mechanics by the user may then cause a field of view of the camera to be changed together with an angulation of the intermediate blade portion. The adjustment joints may be hinges, and the mechanics may comprise an electronic drive.

It can also be preferred that the laryngoscope comprises camera control means configured to orient the camera independently of an adjustment of the blade upon operation of the camera control means by the user. Furthermore, it may be preferred that the laryngoscope comprises at least one further camera, wherein also the at least one further camera is arranged for imaging at least a part of the pharyngeal space from a position on the blade when the blade extends into the pharyngeal space. The at least one further camera may be formed or arranged like the previously described camera, optionally with the exception that the at least one further camera may be arranged more proximally. All of this can further increase the views obtainable from the pharyngeal space and thereby allow for a safer laryngoscopy and subsequent intubation.

The laryngoscope can be made of metal (including alloys) or plastic, particularly hard plastic, for instance. Since the laryngoscope is preferentially reusable, its disinfection after each use may be desired. To alleviate possible problems with the disinfection of the laryngoscope, it may be preferred to use the laryngoscope together with a cover, wherein the cover may be a single-use cover. The cover may be adapted to cover the blade and preferably also the handle including an actuating element, or even all parts, of the laryngoscope, and may be made of a plastic, particularly a soft plastic. For each use of the laryngoscope, a new cover may be slipped over the blade, wherein after the use the cover may be removed from the blade and disposed. Optionally, still a disinfection of the laryngoscope may be carried out. The cover may be formed as a tube or sleeve with a single opening, and may preferably be adapted in its form to a shape of the laryngoscope. In particular a diameter of the tube or sleeve may be adapted to a shape of the laryngoscope, such as particularly to a size of the blade of the laryngoscope in a direction perpendicular to the proximal-distal direction. In particular, the cover is preferentially flexible enough to allow for an unrestricted mobility of the blade. A curvature of a tube or sleeve forming the cover may be adapted to the shape of the laryngoscope particularly in that the tube or sleeve may follow the transition or angle between the blade and the handle in a usage position of the laryngoscope.

The present disclosure includes a use of the laryngoscope for laryngoscopy, in particular for assisting in endotracheal intubation. It may be preferred that the laryngoscope is used with a cover as described above.

These and other aspects of the invention will be apparent from and elucidated with reference to the embodiments described hereinafter.

### BRIEF DESCRIPTION OF THE DRAWINGS

Fig. 1A illustrates schematically and exemplarily an embodiment of a laryngoscope in a usage position with non-operated mechanics for adjusting a blade tip of the laryngoscope,
Fig. 1B illustrates schematically and exemplarily the embodiment of Fig. 1A in a usage position with operated mechanics and with an accordingly adjusted blade tip,
Figs. 2A and 2B illustrate schematically and exemplarily a use of a laryngoscope according to the embodiment of Figs. 1A and 1B,
Fig. 3 illustrates schematically and exemplarily a further embodiment of a laryngoscope in a usage position with different operation states of a mechanics for adjusting a blade tip of the laryngoscope,
Fig. 4 illustrates schematically and exemplarily a measurement of an angle indicating a range of angular motion of a blade of the laryngoscopes, and
Fig. 5 illustrates schematically and exemplarily a use of a cover for the laryngoscope.

### DETAILED DESCRIPTION OF EMBODIMENTS

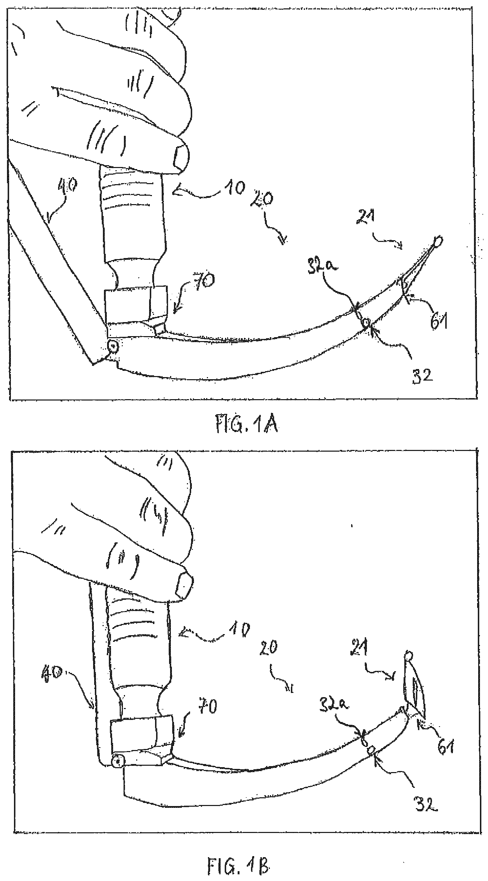

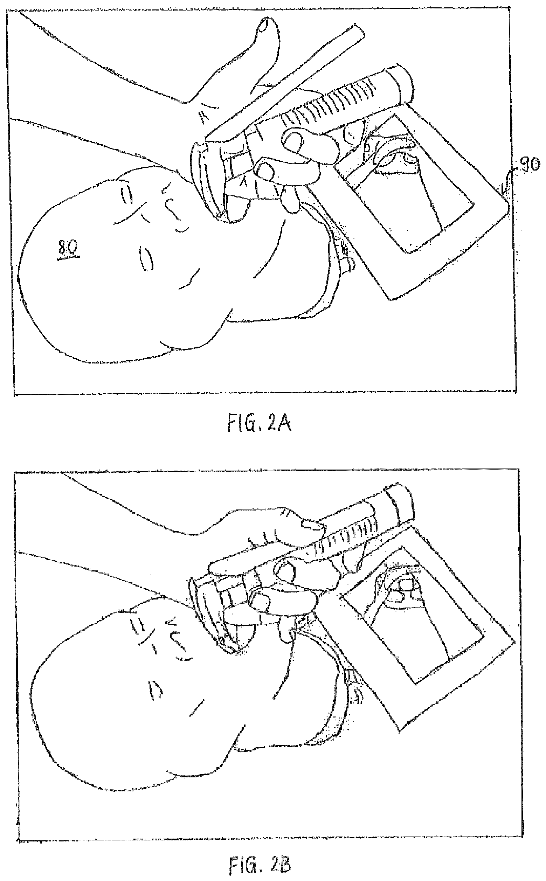

Fig. 1A illustrates schematically and exemplarily a first embodiment of a laryngoscope for assisting a user in endotracheal intubation of a patient by laryngoscopy. The laryngoscope comprises a handle 10 for the user to hold the laryngoscope, and a blade 20 extending from an end portion of the handle 10 in an arc shape. The blade 20 comprises an adjustable blade tip 21 corresponding to a distal end portion of the blade 20 which is connected to a remaining proximal portion of the blade 20 via an adjustment joint 61, wherein the adjustment joint 61 allows the blade tip 21 to be adjusted in its orientation relative to the remaining proximal portion of the blade 20. The adjustment of the blade tip 21 can be controlled by a user holding the laryngoscope via the mechanics of the laryngoscope, which in this embodiment are substantially arranged internally and therefore cannot be seen in Fig. 1A. For enabling a user to operate the mechanics and thus adjust the blade tip 21 while the user holds the laryngoscope, the laryngoscope of this embodiment comprises an actuating element 40 in the form of a lever, wherein the lever 40 connects to a transition between the handle 10 and the blade 20. This could also be viewed as the lever 40 being partially connected to the handle 10 and partially connected to the blade 20. As seen in Fig. 1A, a user may hold the laryngoscope with one hand, with the thumb of the hand resting on the lever 40 and the remaining fingers of the hand resting on the handle 20. In Fig. 1A, an anatomy of the patient is not shown, but the laryngoscope is oriented and held in a way similar to how it would be oriented and held during an actual laryngoscopy. During actual laryngoscopy, the blade 20 would then extend from the handle 10 into a pharyngeal space of the patient, wherein by pressing the lever 40 towards the handle 10, the user would be able to adjust the blade tip 21 and thereby, for instance, apply pressure on the hyoepiglottic ligament to allow for or improve a direct view by the user onto the glottis. The laryngoscope of Fig. 1A in a state with pressed lever 40 and thus adjusted blade tip 21 is shown in Fig. 1B. An operation of the laryngoscope as it would be carried out for laryngoscopy is further illustrated in Figs. 2A and 2B, which are described further below.

To provide the user with further visual information about the pharyngeal space and thus further facilitate intubation, the laryngoscope also comprises an endoscope camera with a camera head 32 for imaging at least a portion of the pharyngeal space from a position on the blade 20 when the blade extends into the pharyngeal space. In this embodiment, the camera head 32 is arranged in a distal half of the blade 20, but proximal with respect to the adjustable tip 21. It will be understood that terms like "proximal(ly)" and "distal(ly)" are used herein to indicate relative positions with respect to a body of the laryngoscope and preferentially not a patient's anatomy. In the embodiment shown, the camera head 32 comprises a lens and a digital image sensor below the lens from which electrical signals are forwarded via a cable which is not visible in Fig. 1A because it may be hidden in an interior of the laryngoscope and particularly the blade 20.

The laryngoscope also comprises illumination means for illuminating the portion of the pharyngeal space to be imaged. In the embodiment shown, the illumination means comprise an LED light source 32a arranged at substantially the same position as the camera head 32. The LED light source 32a is supplied with power by an electric cable which is not visible in Fig. 1A.

While not seen in Figs. 1A and 1B, the mechanics of the laryngoscope connects the lever 40, which itself acts as a mechanical actuating element in this case, to the blade tip 21 via mechanical connection elements. The mechanics with the mechanical connection elements can be realised as in known laryngoscopes of the McCoy type, for instance. It is however also possible to construct the mechanism differently. In particular, instead of a mechanical actuating element like the lever 40 and/or mechanical connection elements, the laryngoscope may comprise an electronic actuating element and/or mechanics being electromechanics comprising at least one electric motor connected to the actuating element and configured to effect a motion of the blade tip 21. Also by operation of such other mechanisms the user can be allowed to determine a position, or orientation, of the adjustment joint 61 and thus a relative angulation between the blade tip 21, forming a distal end portion of the blade 20, and a remaining proximal portion of the blade 20.

As seen in Fig. 1B, the blade tip 21 is adjustable in a direction of the handle 22 to a relatively large degree. Generally, it may be preferred that that the blade tip 21 is adjustable in a direction of the handle 10 by an angle between 0° and 70° relative to a proximal part of the blade 20 which is closest to the blade tip 21. The angle may be measured at the adjustment joint 61, wherein the angle may correspond to an absolute difference between a) an angle of the blade tip 21 in a non-adjusted state, with non-actuated or non-operated actuating element and thus the blade tip 21 substantially aligning with the proximal part of the blade 20 closest to it, and b) an angle of the blade tip 21 with maximally adjusted blade tip 21, with fully actuated or fully operated actuating element.

The laryngoscope of the first embodiment shown in Figs. 1A and 1B also comprises a base joint 70 at a proximal end of the blade, i.e., at the transition between the handle 10 and the blade 20. Via the base joint 70, the blade 20 can be pivoted between a storage position and a usage position, wherein the blade end 20 forms a more obtuse angle to the handle in the usage position than in the storage position. Figs. 1A and 1B show the blade 20 in the usage position. For storing the laryngoscope, it may be preferred to pivot the blade 70 towards the handle 10 about the base joint 70. The base joint 70 may comprise a lock mechanism for preventing a pivoting motion of the blade 20 during use of the laryngoscope, i.e., during laryngoscopy. The lock mechanism may be activated or deactivated, i.e., unlocked, by the user. Alternatively, the base joint 70 may be stiff enough to prevent the pivoting motion during laryngoscopy, but not too stiff to prevent the user from willingly effecting the pivoting motion for storing the laryngoscope with reasonable effort.

Figs. 2A and 2B show schematically and exemplarily an operation of the laryngoscope according to the above described embodiment. For demonstration, an airway manikin 80 is used, and the endoscope camera is connected to a tablet monitor 90 displaying images acquired using the camera. In Fig. 2A, the lever 40 is pressed and thus the mechanics for adjusting the blade tip 21 are not actuated, or operated. Accordingly, the view of the glottis is severely restricted even in the image acquired by the camera. Only arytenoid cartilage is visible, but no vocal cords. On the other hand, in Fig. 2B, the lever 40 is pressed and thus the blade tip 21 is angulated via the mechanism of the laryngoscope. In this way, pressure is applied to the hyoepiglottic ligament at a more favourable angle, thereby improving the upward movement of the epiglottis and the view into the pharyngeal space including the epiglottis and the glottis. In the image seen on the monitor 90, a large portion of the vocal cords are now visible, which would allow for a safe intubation in a subsequent step.

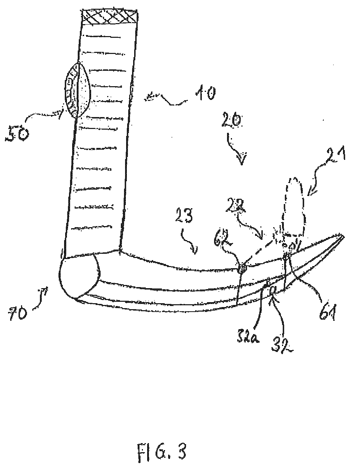

Fig. 3 illustrates schematically and exemplarily a second embodiment of a laryngoscope for assisting a user in endotracheal intubation of a patient by laryngoscopy. Also in this embodiment, the laryngoscope comprises a handle 10 for the user to hold the laryngoscope, a blade 20 adapted to extend from the handle 10 into a pharyngeal space of the patient when the laryngoscope is held by the user, and a camera 32 for imaging at least a portion of the pharyngeal space from a position on the blade 20 when the blade 20 extends into the pharyngeal space. Furthermore, the laryngoscope again comprises mechanics operable by the user while holding the laryngoscope, wherein the blade 20 comprises an adjustable blade tip 21, the blade tip 21 being adjustable by the user via the mechanics. The laryngoscope of the second embodiment also comprises a base joint 70 as discussed above, for pivoting the blade 20 between a usage position, as seen in the Figs., and a storage position in which the blade 10 forms a less obtuse angle with the handle 10 or even abuts the handle 10.

In the second embodiment, however, the actuating element is an adjustment wheel 50 which the user can rotate with, for instance, the thumb while holding the handle 10. A rotation of the adjustment wheel 50 may translate into an adjustment of the blade tip 21. The translation may be realized, for instance, by an electronic signal triggered by the rotation, which is forwarded to one or more electric motors.

Furthermore, the second embodiment differs from the first embodiment in that the laryngoscope comprises not only a single adjustment joint, but instead two adjustment joints 61, 62, wherein a first adjustment joint 61 of the two is arranged at a first distal position of the blade 20 and a second adjustment joint 62 of the two adjustment joints is arranged at a second distal position of the blade 20, wherein the second distal position is more proximal than the first distal position. Thus, when understanding a most distal section of the blade 20, i.e., the section distal to the first angulation joint 61, as blade tip 21, the second angulation joint 62 now divides a region of the blade 20 located proximally to the blade tip 21 into two sections 22, 23 adjacent to the second angulation joint 62. The mechanics of the laryngoscope according to the second embodiment are operable by the user so as to adjust a shape of the blade 20 via a position of the at least one further adjustment joint 62, particularly by tilting the sections 22, 23 with respect to each other. The blade tip 21 may still be adjustable separately, i.e., as described above with respect to the first embodiment. Thus, the blade tip 21 may be angulated with respect to the blade section 22 between the first angulation joint 61 and the second angulation joint 62, and the blade section 22 may be angulated with respect to a remaining proximal portion 23 of the blade. Both angles may have a similar range. Hence, also the two sections 22, 23 adjacent to the second angulation joint 62 may be tilted with respect to each other by an angle between 0° and 70°. The angle may be measured analogously as described above for the adjustment of the blade tip 21 in the case of a single adjustment joint 61.

Fig. 3 shows the blade, particularly a distal portion thereof, both in a non-adjusted state (solid contours) and in an adjusted state (dashed contours). It can be seen that, in the adjusted state, an angulation occurs both at the first adjustment joint 61 and at the second adjustment joint 62. Preferably, operation of the mechanics of the laryngoscope causes both adjustment joints 61, 62 to be jointly adjusted so that the blade tip 21 and the section 22 between the adjustment joints 61, 62, which is immediately proximal to the blade tip 61, are simultaneously angled towards the handle 10 by a respective adjustment angle. As mentioned above, both adjustment angles may range between 0° and 70°. While in Fig. 3 the second adjustment joint 62 is still arranged in a distal half of the blade 20, it may more generally be preferred that the adjustment joint 62 and optional further adjustment joints are arranged in a middle or middle to proximal region of the blade 20.

The camera head 32 is, in the second embodiment, arranged distally from the second adjustment joint 62 on the blade 20. In this way, as illustrated in Fig. 3 by a moved copy of the camera head 32 on the adjusted copy of the distal blade portion having dashed contours, an orientation, or viewing angle, of the camera head 32 can be changed together with an adjustment of the blade 20 by operation of the mechanics. Generally, however, the camera head 32 may also be orientable independently from an adjustment of the blade 20 and particularly the blade tip 21. For this purpose, the laryngoscope may comprise camera control means configured to orient the camera head 32 independently of an adjustment of the blade 20 upon operation of the camera control means by the user. For instance, the camera head 32 may be controlled electromechanically via a cable as shown in Figs. 1A and 1B.

While not shown, it may also be preferred that the laryngoscope comprises at least one further camera, wherein also the at least one further camera is arranged for imaging at least a part of the pharyngeal space from a position of the blade 20 when the blade 20 extends into the pharyngeal space. The at least one further camera or its camera head may be arranged on the blade 20 more proximally than the camera heads 32 of the first and the second embodiment described above.

The above embodiments of a laryngoscope allow to adapt the blade to each patient and each clinical situation, thus enabling an optimal pressure axis and an optimal pressure angle for effecting the desired movement of the epiglottis for a respective patient during a respective laryngoscopy. This can be achieved by a flexible adjustment of the blade curvature via for instance, one or more adjustment joints and a manual and/or electronic mechanism. In particular, a lever mechanism or a Bowden cable mechanism may be used. Additional degrees of freedom can be beneficial, such as by adding adjustment joints. Moreover, an optimal position of the camera can improve the view into the pharyngeal space. The camera may move with the blade, or spatula, and/or may be moved by itself to adjust a camera viewing angle and thereby further improve the view into the pharyngeal space. The camera may be a commercial endoscope camera such as the DEPSTECH 3.9 mm WiFi endoscope camera with light mobile phone endoscope 1080P HD inspection camera, for instance. Otherwise, the laryngoscope may substantially correspond to a commercial McCoy lever laryngoscope as manufactured by the company Penlon Ltd., for instance. The laryngoscope may also be manufactured using a 3D printer, with an added commercial endoscope camera.

Historically, the Macintosh laryngoscope was used for laryngoscopy, and in particular also for the pressure application on the hyoepiglottic ligament. The McCoy laryngoscope was widely adopted as a modification of the Macintosh laryngoscope with which, by virtue of the lever mechanism to effect blade tip movement, the pressure application on the hyoepiglottic ligament and thereby the view on the glottis could be improved. However, since the introduction of videolaryngoscopy, the lever mechanism was no longer considered important, since the indirect view corresponding to the camera images was considered sufficient. It has now been realised by the inventors, however, that the ability to adjust the blade curvature and particularly the blade tip cannot only improve a direct view into the pharyngeal space, but also the view captured by the camera images. A laryngoscope as disclosed herewith could also be referred to as an adjustable-tip-videolaryngoscope, or ATV in short.

More preferably, the laryngoscope as disclosed herewith could be referred to as a dynamically adjustable videolaryngoscope for endotracheal intubation, or DAVE in short.

The embodiments disclosed herein gradually differ from each other in a safety for the patient, an ease of handling by the user, a learning curve for users to learn how to operate the respective laryngoscope and a constructional complexity, i.e., technical demands for a realisation and manufacturing of a respective laryngoscope. However, all embodiments have been seen to allow to meet all of the mentioned requirements to a sufficient degree. Particularly relevant features of the laryngoscope in this regard include the positioning of the camera, the one or more angulation points, a relation between the camera position and the one or more angulation points, a range of angular motion of the blade tip and the optional further adjustable blade sections, the number of angulation points and the type of mechanism.

For instance, regarding the range of angular motion at a respective angulation point, a range from 0° to 70° as outlined above may be possible, but also a range from 0° to 80° could be conceived. On the other hand, also a range from 30° to 60° can be preferred. Thus, in particular, the blade tip may also be adjustable in the direction of the handle by an angle between 30° and 60° relative to the proximal part of the blade which is closest to the blade tip. When the mechanism of the laryngoscope is not operated, the blade tip may then not be aligned with a remaining portion of the blade, but may be angulated by 30° with respect to the remaining portion of the blade.

As to the relative positioning of the camera head and the angulation point for the blade tip, two kinds of embodiments may be distinguished which may have different advantages. In a first kind of embodiments, the camera head may be positioned on the blade proximally, i.e. at a smaller distance from the handle in a usage position of the blade, from the point at which the blade tip is angulated with respect to a remaining proximal portion of the blade, such as via a most distal angulation joint. In a second kind of embodiments, the camera head may be positioned on the blade distally from the point at which the blade tip is angulated with respect to the remaining proximal portion of the blade. Thus, in the second kind of embodiments, the camera head may be positioned on the blade tip, allowing the camera head to move with the blade tip upon actuation of the mechanics of the laryngoscope by the user. Illumination means such as an LED may be positioned like, such as together with, the camera head.

Two or more angulation points may be provided to enable a more flexible adaptation of the blade curvature to a patient anatomy. The multiple blade sections created in this way may be angulated jointly. In other words, a single user operation may cause the mechanism to adjust an angular position of each of the sections simultaneously.

Different sub-ranges of the range of angular motion of the blade tip and optional further proximal blade sections may be identified by their relevance for respective different stages of laryngoscopy. As mentioned above, a total range of angular motion may be 0° to 70° or 80°. Also 0° to 60° can be sufficient. In an angular sub-range from 0° to 20°, the blade may be considered to be essentially straight and the epiglottis may be loaded. In an angular sub-range from 20° to 40°, the blade shape may correspond to that of a Macintosh laryngoscope, and the blade tip may be placed above the epiglottis, wherein a direct view on the glottis may still be possible. In an angular sub-range above 40°, the blade may be referred to as having a hyperangulated shape, which can optimise the camera view at the cost of possibly rendering a direct view on the glottis no longer possible.

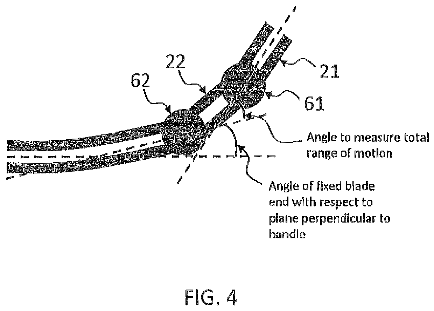

The above angles indicating the angular motion may be measured between a) a direction into which the blade tip points when the mechanics of the laryngoscope are operated, particularly maximally operated, and b) a direction into which a non-actuated blade portion points, wherein the non-actuated blade portion is a portion of the blade which remains fixed when the user actuates the mechanism of the laryngoscope. The non-actuated blade portion may correspond to a blade portion proximal to a most proximal angulation point, which may itself be bent away out of a plane perpendicular to a handle of the laryngoscope in a usage position due to an intrinsic curvature of the blade. This is schematically and exemplarily illustrated in Fig. 4.

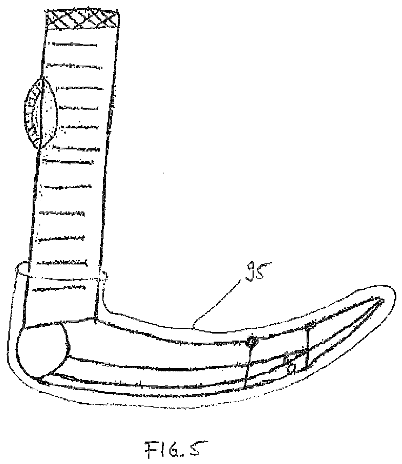

Fig. 5 illustrates schematically and exemplarily a use of a cover 95 for a laryngoscope according to the second embodiment described above. The laryngoscope is shown again in its usage position, although, in contrast to Fig. 3, only in its state without adjusted tip, which may also be referred to as relaxed state. The tube-like or sleeve-like cover 95 substantially conforms to the blade and a part of the handle of the laryngoscope. A space between the laryngoscope and the cover 95 is exaggerated in Fig. 5 for clarity. In reality, the cover 95, which may be made of a flexible material, may fit more tightly around a body of the laryngoscope. The cover 95 could similarly fit around a laryngoscope according to the first embodiment described above. Irrespective of the embodiment, the cover 95, leading with its single opening, may be pulled, pushed and/or rolled over the laryngoscope in a direction from the blade tip towards the handle for covering the laryngoscope, and pulled, pushed and/or unrolled from the laryngoscope in a direction from the handle towards the blade tip for uncovering the laryngoscope. A cover as shown in Fig. 5 could be used as a single-use part, i.e., may be replaced by a new cover for each laryngoscopy to be conducted. In this way, disinfection demands for the laryngoscope itself, which may be reused over a plurality of laryngoscopies, may be reduced.

Previously, Macintosh blades with a fixed blade tip angle of 30° (Macintosh videolaryngoscopy) and hyperangulated blades with a fixed blade tip angle up to an angle of 60° (hyperangulated video laryngoscopy) have been used for videolaryngoscopy. While Macintosh videolaryngoscopy allows for a direct view of the glottis in addition to the indirect camera view (optional videolaryngoscopy), hyperangulated videolaryngoscopy allows for an improved indirect camera view, but often only at the cost that a direct view of the glottis is no longer possible (obligatory videolaryngoscopy). Moreover, while the larger blade curvature and particularly the larger angulation of the blade tip in hyperangulated videolaryngoscopy can allow for an improved pressure application on the hyoepiglottic ligament to moderate the upward movement of the epiglottis, it could be shown that the increase in visibility of the glottis normally expected from this is not necessarily accompanied by an improvement in intubation conditions. Both Macintosh videolaryngoscopy and hyperangulated videolaryngoscopy can therefore be preferred over each other depending on the context. For many patients, Macintosh videolaryngoscopy is preferred for enabling endotracheal intubation, but hyperangulated videolaryngoscopy is often seen as a fallback/rescue procedure in case Macintosh videolaryngoscopy is considered difficult. Indeed, switching from one blade shape to another has been shown to be useful and beneficial in individual patients with difficult airways. Switching between blades, however, is generally undesirable, since it can interrupt a workflow of the laryngoscopy. This can be avoided with a videolaryngoscope comprising an adjustable blade shape that can be flexibly adapted to a patients anatomical needs. Embodiments of a laryngoscope of this type are disclosed herein.

Videolaryngoscopy has been demonstrated to have a higher efficacy profile and be safer for patients. With approximately 320 million general anaesthesias being performed worldwide of which many are intubation anaesthesias, this has a high clinical value. However, for some patients, videolaryngoscopy is still difficult. Particularly patients with relevant visual obstructions to the glottis are still at risk. Adjustable videolaryngoscopes for endotracheal intubations (DAVEs) as disclosed herein allow for better clinical outcomes for these patients in particular. But, individualised, flexibly adapted videolaryngoscopy using adjustable-tip videolaryngoscopes as disclosed herein can also be beneficial for other patients since, for instance, this allows avoiding unnecessary multiple intubation attempts and/or carrying out the laryngoscopy less invasively.

Disclosed herewith are embodiments of a laryngoscope configured to adapt its blade curvature individually to a patient's anatomy and thus, in combination with a camera arranged on the blade, to achieve an optimised view of the glottis so that endotracheal intubation can be performed optimally and as atraumatically as possible.

The advantages of a laryngoscope as disclosed herein have been demonstrated in a study conducted with 18 anaesthetists and nurse practitioners experienced and specialized in laryngoscopy as participants, and using three different airway manikins. The participants were pre-trained and familiarized with the manikins, whereafter they were asked to intubate the manikins in a randomized sequence, either starting with direct levering laryngoscopy (naked eye, direct view on the glottis with monitored camera images concealed) or camera-assisted DAVE (camera images on the monitor not concealed), and starting from four different predefined base line conditions corresponding to glottis view grades 2a, 2b, 2c and 3 as gathered from the camera images according to the six-staged modified Cormack-Lehane grading scheme disclosed, for instance, in Table 1 of the article "Hyperangulated blades or direct epiglottis lifting to optimize glottis visualization in difficult Macintosh videolaryngoscopy: a non-inferiority analysis of a prospective observational study" by V. Wünsch et al., Frontiers in Medicine, volume 10 (2023). The same grading scheme was then used by the participants to quantify both the direct (naked eye) as well as the indirect (camera) glottis view before and after a respective epiglottis elevation attempt. Moreover, respective percentages of the glottic opening (POGO) score before and after epiglottis elevation attempts were recorded based both on the direct and the indirect view.

From a statistical analysis of the data acquired in the study it could be seen that both the POGO scores and the glottis view grades relevantly improved due to the elevation mechanisms of the epiglottis and also by using an endoscope camera mounted at the distal half of the blade. For direct laryngoscopic glottis visualization (naked eye), epiglottis elevation by the hinged tip led to approximately 44 % improved view grades with a mean view grade improvement of approximately 0.5 and a mean POGO improvement of approximately 10 %. For videolaryngoscopic glottis visualization (as displayed on the screen), epiglottis elevation by the hinged tip led to approximately 96 % improved view grades with a mean view grade improvement of approximately 1.5 and a mean POGO improvement of approximately 36 %. Hence, the elevation mechanism was more effective in conjunction with videolaryngoscopy than with direct laryngoscopy. Particularly in severely restricted glottis view conditions the elevation mechanism was very effective in conjunction with videolaryngoscopy (grade 2c view + 51 % and grade 3 view + 44 % POGO improvement approximately) but less effective in conjunction with direct laryngoscopy (grade 2c view + 10 % and grade 3 view + 5 % POGO improvement approximately). The mounted camera at the distal half of the blade improved the glottis visualization in approximately 28 % (61/216) of the applications without hinged tip and in approximately 87 % (187/216) of the applications with hinged tip. The mean glottis grade improved by approximately 0.8 grades and POGO by approximately 27 %. The data indicate an additive synergistic effect between the additional camera and glottis elevation mechanism, as the effect of the camera was particularly high if the tip of the blade was hinged (approximately 40 % POGO improvement versus 14 % without hinged tip and approximately 1.3 grades glottis view improvement versus 0.3 grades without hinged tip).

Although in the above embodiments only particular examples for the mechanics effecting the angulation of the blade tip and optionally further segments of the blade have been mentioned, other types of mechanics are possible. In particular, mechanics not involving elements such as Bowden cables or electric motors could be employed. Also other actuating elements than those mentioned above could be used. Generally, the mechanics can include mechanical and/or electric parts or elements, i.e., also combinations mechanical and electric elements. The mechanics of the laryngoscope could also be referred to as a mechanism.

Moreover, while above an embodiment was described in which the blade comprises an adjustable blade tip and further adjustable blade sections, the blade may even comprise a blade tip that itself comprises two or more blade tip sections, which are separated from and can be angulated with respect to each other via respective one or more angulation joints arranged on the blade tip.

The blade of the laryngoscope may also be referred to as a spatula.

Other variations to the disclosed embodiments can be understood and effected by those skilled in the art in practicing the claimed invention, from a study of the drawings, the disclosure and the appended claims.

In the claims, the word "comprising" does not exclude other elements or steps, and the indefinite article "a" or "an" does not exclude a plurality.

A single element may fulfil the functions of several elements recited in the claims and vice versa. The mere fact that certain measures are recited in mutually different dependent claims does not indicate that a combination of these measures cannot be used to advantage.

Any reference signs in the claims should not be construed as limiting the scope.

The invention relates to a laryngoscope for assisting a user in endotracheal intubation of a patient by laryngoscopy. The laryngoscope comprises a) a handle for the user to hold the laryngoscope, b) a blade adapted to extend from the handle into a pharyngeal space of the patient when the laryngoscope is held by the user, and c) a camera for imaging at least a portion of the pharyngeal space from a position on the blade when the blade extends into the pharyngeal space. The laryngoscope further comprises mechanics operable by the user while holding the laryngoscope, and the blade comprises an adjustable blade tip, wherein the blade tip is adjustable by the user via the mechanics. A laryngoscope of this kind allows for safer endotracheal intubations.

## Claims

1. A laryngoscope for assisting a user in endotracheal intubation of a patient by laryngoscopy, the laryngoscope comprising:
- a handle (10) for the user to hold the laryngoscope,
- a blade (20) adapted to extend from the handle (10) into a pharyngeal space of the patient when the laryngoscope is held by the user, and
- a camera (32) for imaging at least a portion of the pharyngeal space from a position on the blade (20) when the blade (20) extends into the pharyngeal space,
wherein the laryngoscope further comprises mechanics operable by the user while holding the laryngoscope, and the blade (20) comprises an adjustable blade tip (21), wherein the blade tip (21) is adjustable by the user via the mechanics.

2. The laryngoscope according to claim 1, further **characterised in that** the laryngoscope comprises an actuating element (40; 50) on the handle (10) or on a transition between the handle (10) and the blade (20), by which the user can control the mechanics and thus adjust the blade tip (21) via one or more adjustment joints (61, 62).

3. The laryngoscope according to claim 2, further **characterised in that** the actuating element (40; 50) is a mechanical actuating element.

4. The laryngoscope according to claim 2 or 3, further **characterised in that** the mechanics connect the actuating element (40; 50) to the blade tip (21) via mechanical connection elements.

5. The laryngoscope according to claim 2, further **characterised in that** the actuating element is an electronic actuating element.

6. The laryngoscope according to claim 5, further **characterised in that** the mechanics are electromechanics comprising at least one electric motor electronically connected to the actuating element.

7. The laryngoscope according to any of the preceding claims, further **characterised in that** the blade tip (21) is adjustable in a direction of the handle (10) by an angle between 0° and 70° relative to a proximal part of the blade (20) which is closest to the blade tip (21).

8. The laryngoscope according to any one of the preceding claims, further **characterised in that** the blade tip (21) forms a distal end portion of the blade (20) and the blade (20) comprises an adjustment joint (61) which is arranged proximally at the distal end portion and whose position is determined through the mechanics operable by the user.

9. The laryngoscope according to claim 8, further **characterised in that** the blade (20) has at least one further adjustment joint (62), which divides a region of the blade (20) located proximally of the blade tip (21) into two or more sections (22, 23) adjacent to the respective further adjustment joint (62), wherein the mechanics operable by the user are configured to further adjust a shape of the blade (20) via a position of the at least one further adjustment joint (62).

10. The laryngoscope according to claim 9, further **characterised in that** the at least one further adjustment joint (62) is configured to tilt the respective two adjacent sections (22, 23) of the blade (20) relative to each other by an angle between 0° and 70°.

11. The laryngoscope according to claim 10, further **characterised in that** operation of the mechanics causes the two or more adjustment joints (61, 62) to be jointly adjusted so that the blade tip (21) and one or more sections (22) of the blade (20) proximal to the blade tip (21) are simultaneously angled towards the handle (10) by a respective adjustment angle.

12. The laryngoscope according to any of claims 9 to 11, further **characterised in that** the at least one further adjustment joint (62) is arranged in a middle to proximal region of the blade (20).

13. The laryngoscope according to any of claims 8 to 12, further **characterised in that** the camera (32) is arranged on the blade tip (21) or, insofar as the laryngoscope is a laryngoscope according to any of claims 9 to 12, **in that** the camera is arranged at least distally from the at least one further adjustment joint (62) on the blade (20), so that an orientation of the camera (32) can be changed together with an adjustment of the blade (20) by operation of the mechanics.

14. The laryngoscope according to any of the preceding claims, further **characterised in that** it comprises camera control means configured to orient the camera (32) independently of an adjustment of the blade (20) upon operation of the camera control means by the user.

15. The laryngoscope according to any of the preceding claims, further **characterised in that** it comprises at least one further camera, wherein also the at least one further camera is arranged for imaging at least a part of the pharyngeal space from a position on the blade (20) when the blade (20) extends into the pharyngeal space.
